# EUROPEAN PATENT SPECIFICATION

(11) **EP 3 417 886 B1**
(45) Date of publication and mention of the grant of the patent: **16.03.2022**
(21) Application number: 18181496.3
(22) Date of filing: 22.10.2012
(51) Int. Cl.: A61L 27/26, A61L 27/50

(54) **COLLAGEN-POLYSACCHARIDE MATERIALS MIMICKING BLOOD VESSELS, TISSUES AND BONES**
BLUTGEFÄSSEN, GEWEBE UND KNOCHEN IMITIERENDE KOLLAGEN-POLYSACCHARID-MATERIALIEN
MATÉRIAUX À BASE DE COLLAGÈNE-POLYSACCHARIDE IMITANT LES VAISSEAUX SANGUINS, LES TISSUS ET LES OS

(30) Priority: 21.10.2011 US 201161550104 P
(43) Date of publication of application: 26.12.2018
(62) Divisional of application: 12842642.6
(73) Proprietor: Viscofan Collagen USA Inc., Bridgewater, NJ 08807 (US)
(72) Inventor: Battersby, Richard E., Frenchtown, NJ 08825 (US); Goldfarb, Eugene, Marlboro, NJ 07746 (US); Mathews, David E., Somerville, NJ 08876 (US); Shedlock, Michael T., Easton, PA 18045 (US); Guzman, Norberto A., East Brunswick, NJ 08816 (US)
(74) Representative: Herrero & Asociados, S.L.

(56) References cited:
- CN-A- 101 845 226
- US-A- 4 182 054
- US-A1- 2001 014 662
- US-A1- 2005 031 741
- US-A1- 2008 107 744
- US-B1- 6 235 328

## Description

### Field of the Invention

The present invention relates to a collagen-polysaccharide material which may be in the form of a hollow tube that mimics a blood vessel, a sheet that mimics a tissue, or a solid that mimics a bone. It is disclosed herein that these materials are intended for use in medical, pharmaceutical and orthopedic applications. Processes for producing said materials are also disclosed, as well as the use of these materials in testing phlebotomical, surgical or orthopedic instrumentation or practising phlebotomical, surgical or orthopedic procedures.

### Discussion of the Background

Collagen is a major fibrous protein constituent of cartilage, skin, bones, tendons, and other connective tissues of animals, as well as of skin, bones and scales of aquatic animals.

Collagen is present in all types of multicellular organisms and is probably the most abundant animal protein in nature. Collagen is located in the extracellular matrix of connective tissues and contributes to tissue integrity and mechanical properties.

Collagen molecules are composed of three polypeptide chains called alpha chains. The alpha chains contain about 1000 amino acid residues and, with the exception of short sequences at the ends of the chains, every third amino acid in each chain is glycine. The molecular formula of an alpha chain can thus be approximated as (X-Y-Gly)₃₃₃, where X and Y represent amino acids other than glycine. In collagen from mammals and birds about 100 of the X positions are proline, and about 100 of the Y positions are hydroxyproline. Collagen molecules have been classified into at least 29 types in the order in which they were purified and characterized, and grouped into classes according to their physicochemical properties.

Due to their excellent biocompatibility, biodegradability, and ease of extraction, purification and processing, collagen molecules have found use as a versatile biomaterial in numerous medical and pharmaceutical applications. Furthermore, collagen is used in the food and beverage industry, cosmetic industry, and nutraceutical industry in a variety of compositions.

Unembalmed anatomical materials simulating a "real world" tissue for testing phlebotomical, surgical or orthopedic instrumentation during research and development and/or for practicing phlebotomical, surgical or orthopedic procedures during the training of healthcare professionals (e.g., physicians, phlebotomists, surgeons, and medical students) suffer from a number of disadvantages including but not limited to a short shelf-life, rapid decomposition and the emission of foul odors, as well as the reliance on obtaining the unembalmed anatomical materials from animals and human cadavers. Artificial blood vessels, tissues and bone are know. See for example US2001/014662A1 which discloses an artificial bone material comprising collagen and cellulose.

Accordingly, there remains a critical need for a medical device in the form of an artificial blood vessel, an artificial tissue, and/or an artificial bone that is manufactured to replicate a naturally occurring blood vessel, tissue, and bone for testing phlebotomical, surgical or orthopedic instrumentation during research and development and/or for practicing phlebotomical, surgical or orthopedic procedures during the training of healthcare professionals (e.g., physicians, phlebotomists, surgeons, and medical students) while obviating the above-identified problems.

### SUMMARY OF THE INVENTION

The present invention provides a collagen-cellulose material comprising: 1.0 to 9.0 wt. % of a collagen, based on a total weight of the collagen-cellulose material; 0.2-3.0 wt.% of cellulose or a derivative thereof, based on the total weight of the collagen-cellulose material; 0.5-6.5 wt.% of at least one acid selected from the group consisting of an inorganic acid, an organic acid, and mixtures thereof, based on the total weight of the collagen-cellulose material; and water, wherein the collagen-cellulose material is in a form of an artificial blood vessel having an internal and/or external diameter including from 0.1 to 50.0 mm, an artificial tissue, and/or an artificial bone.

An exemplary aspect of the present invention is to provide a collagen-cellulose material comprising: a collagen; cellulose or a derivative thereof; at least one acid selected from an inorganic acid, an organic acid, and mixtures thereof; and water.

An exemplary aspect of the present invention is to provide a collagen-cellulose material comprising: 1.0-9.0 wt. % of a collagen, based on a total weight of the collagen-cellulose material; 0.2-3.0 wt. % of cellulose or a derivative thereof, based on the total weight of the collagen-cellulose material; 0.5-6.5 wt. % of at least one acid selected from an inorganic acid, an organic acid, and mixtures thereof, based on the total weight of the collagen-cellulose material; and water.

An exemplary aspect of the present invention is to provide a collagen-cellulose material comprising: 3.0-7.0 wt. % of a collagen, based on a total weight of the collagen-cellulose material; 0.8-2.0 wt. % of cellulose or a derivative thereof, based on the total weight of the collagen-cellulose material; 1.0-6.0 wt. % of at least one acid selected from an inorganic acid, an organic acid, and mixtures thereof, based on the total weight of the collagen-cellulose material; and water.

An exemplary aspect of the present invention is to provide a collagen-cellulose material comprising: 3.0-7.0 wt. % of a collagen, based on a total weight of the collagen-cellulose material; 0.8-2.0 wt. % of cellulose or a derivative thereof, based on the total weight of the collagen-cellulose material; 1.0-6.0 wt. % of at least one acid selected from an inorganic acid, an organic acid, and mixtures thereof, based on the total weight of the collagen-cellulose material; 0.1-25 wt. % of one or more compounds selected from pectin, sodium citrate, ammonium sulfate, citric acid, a crosslinking agent (e.g., glutaraldehyde and/or hydroxyacetaldehyde), an aluminum salt, a sugar, a sugar derivative obtained from a caramelization process, a sugar mixture comprising a sugar or a derivative thereof and a crosslinking agent (e.g., MAILLOSE^{®}), a browning agent, glycerin, an antimicrobial agent (e.g., sodium benzoate and/or hypochlorite), mineral oil, poly(lactic-co-glycolic acid) (PLGA), hydroxyapatite, a surfactant, a plasticizer, a dye, and mixtures thereof, based on the total weight of the collagen-cellulose material; and water.

The collagen-cellulose material may be in the form of a hollow tube that mimics a blood vessel (e.g., an artery, a vein, or a capillary), a sheet (e.g., a slab, a pad, a membrane, or a film) that mimics a tissue, or a solid that mimics a bone. The hollow tube may have any desired dimension, including any desired length, aspect ratio, inner diameter, and/or outer diameter. The sheet may have any desired dimension, including any desired length, width, and/or thickness. The solid may have any desired dimension, including any desired shape, length, width, thickness, and/or outer diameter. The hollow tube, the sheet, and the solid may also have a desired tensile strength, modulus of elasticity, flexibility, porosity, compressibility, decompressibility, hardness, degree of crosslinking, wetness, sealability, and/or stability.

The foregoing discussion exemplifies certain aspects of the present invention. Additional exemplary aspects of the present invention are discussed in the following detailed description of the invention. The following description is to be regarded as illustrative in nature, and not as restrictive.

### DETAILED DESCRIPTION OF THE INVENTION

Unless specifically defined, all technical and scientific terms used herein have the same meaning as commonly understood by a skilled artisan in the relevant technological field.

The materials, processes and examples described herein are for illustrative purposes only and are therefore not intended to be limiting, unless otherwise specified.

Where a closed or open-ended numerical range is described herein, all values and subranges within or encompassed by the numerical range are to be considered as being specifically included in and belonging to the original disclosure of the present application as if these values and subranges had been explicitly written out in their entirety.

Recitations of "a" or "an" element, article or limitation within the claims and/or preferred embodiments and the specification is appropriately construed, and understood in the context of the present invention, to mean "one or more" unless otherwise specified.

The present invention provides a collagen-cellulose material comprising: a collagen; cellulose or a derivative thereof; at least one acid selected from an inorganic acid, an organic acid, and mixtures thereof; and water.

The present invention provides a collagen-cellulose material comprising: 1.0-9.0 wt. % of a collagen, based on a total weight of the collagen-cellulose material; 0.2-3.0 wt. % of cellulose or a derivative thereof, based on the total weight of the collagen-cellulose material; 0.5-6.5 wt. % of at least one acid selected from an inorganic acid, an organic acid, and mixtures thereof, based on the total weight of the collagen-cellulose material; and water.

The present invention provides a collagen-cellulose material comprising 1.0-9.0 wt. % of a collagen, including for example 1.5-8.5 wt. %, 2.0-8.0 wt. %, 2.5-7.5 wt. %, 3.0-7.0 wt. %, 3.5-6.5 wt. %, 4.0-6.0 wt. %, 4.5-5.5 wt. %, or 5.0 wt. %, based on a total weight of the collagen-cellulose material. The collagen is preferably present in an amount of 4.0-6.0 wt. %, 3.5-5.5 wt. %, or 3.0-5.0 wt. %, based on a total weight of the collagen-cellulose material. The collagen is particularly preferably present in an amount of 3.5-5.5 wt. %, based on a total weight of the collagen-cellulose material.

The present invention provides a collagen-cellulose material comprising 0.2-3.0 wt. % of cellulose or a derivative thereof, including for example 0.4-2.8 wt. %, 0.6-2.6 wt. %, or 0.8-2.4 wt. %, 1.0-2.2 wt. %, 1.2-2.0 wt. %, 1.4-1.8 wt. %, or 1.6 wt. %, based on a total weight of the collagen-cellulose material. The cellulose or the derivative thereof is preferably present in an amount of 0.6-2.2 wt. %, 0.8-2.0 wt. %, 1.0-1.8 wt. %, 1.2-1.6 wt. %, or 1.4 wt. %, based on a total weight of the collagen-cellulose material. The cellulose or the derivative thereof is particularly preferably present in an amount of 0.8-2.0 wt. %, based on a total weight of the collagen-cellulose material.

The cellulose may be obtained/derived from any plant source or material (e.g., wood). A particularly preferred source of cellulose is pectin-rich plant materials including for example peel (especially albedo, the white portion) of citrus fruits (especially lemon, lime, orange, and grapefruit), apple pomace, sugar beet pulp, sunflower heads, carrots, potatoes, tomatoes, and combinations thereof. For example, lemon peel contains approximately 2-4 wt. % of pectin when fresh and 20-40 wt. % of pectin when dried, whereas dried apple pomace contains approximately 10-20 wt. % of pectin. Cellulose obtained/derived from wood and/or orange peel is particularly preferred. Cellulose obtained/derived from orange peel is especially preferred because of a lower viscosity and a higher pectin content relative to that of wood.

The cellulose may exist in one or more various forms including for example powdered cellulose, microcrystalline cellulose, cellulose fibers, microfibrillated cellulose, and mixtures thereof. The microfibrillated cellulose is a nanocellulose material composed of nanosized cellulose fibrils having a high aspect ratio with a typical width of 5-20 nm and a typical length of up to 2,000 nm.

The derivative of cellulose may be an ether derivative of cellulose, non-limiting examples of which include methylcellulose, ethylcellulose, ethylmethylcellulose, hydroxyethylcellulose, hydroxyethylmethylcellulose, hydroxypropylcellulose, hydroxypropylmethylcellulose, ethylhydroxyethylcellulose, carboxymethylcellulose, carboxyethylcellulose, and mixtures thereof.

The derivative of cellulose may be an ester derivative of cellulose, non-limiting examples of which include cellulose acetate, cellulose triacetate, cellulose propionate, cellulose acetate propionate, cellulose acetate butyrate, cellulose nitrate, cellulose sulfate, and mixtures thereof.

The cellulose component of the collagen-cellulose material of the present invention may be partially substituted with, or completely replaced by, one or more polysaccharides other than cellulose. Non-limiting examples of the one or more polysaccharides other than cellulose include alginate, α-glucan, amylopectin, amylose, arabinoxylan, β-glucan, carrageenan, chitin, chitosan, cyclodextrin, dextran, dextrin, fructan, gellan gum, glucan, glycogen, hemicellulose, inulin, maltodextrin, pectin, starch, and xanthan gum. Preferred collagen-polysaccharide materials of the present invention include a collagen-cellulose material, a collagen-alginate material, and mixtures thereof. A collagen-cellulose material represents a particularly preferred collagen-polysaccharide material of the present invention.

The present invention provides a collagen-cellulose material comprising 0.5-6.5 wt. % of at least one acid selected from an inorganic acid, an organic acid, and mixtures thereof, including for example 1.0-6.0 wt. %, 1.5-5.5 wt. %, 2.0-5.0 wt. %, 2.5-4.5 wt. %, 3.0-4.0 wt. %, or 3.5 wt. %, based on a total weight of the collagen-cellulose material. The at least one acid is preferably present in an amount of 1.0-6.0 wt. %, 1.5-5.5 wt. %, 2.0-5.0 wt. %, 2.5-4.5 wt. %, or 3.0-4.0 wt. %, based on a total weight of the collagen-cellulose material. The at least one acid is particularly preferably present in an amount of 2.0-5.0 wt. %, 2.5-4.5 wt. %, or 3.0-4.0 wt. %, based on a total weight of the collagen-cellulose material.

The acid may be one or more inorganic acids. Non-limiting examples of the inorganic acid include hydrochloric acid, nitric acid, phosphoric acid, sulfuric acid, boric acid, hydrofluoric acid, hydrobromic acid, perchloric acid, and mixtures thereof. Preferred inorganic acids include hydrochloric acid, phosphoric acid, sulfuric acid, and mixtures thereof. A particularly preferred inorganic acid is hydrochloric acid.

The acid may be one or more organic acids. Non-limiting examples of the organic acid include formic acid, acetic acid, propionic acid, butyric acid, valeric acid, caproic acid, and mixtures thereof. Preferred organic acids include formic acid, acetic acid, propionic acid, and mixtures thereof. A particularly preferred organic acid is acetic acid.

The acid may be a mixture of one or more inorganic acids and one or more organic acids. A weight ratio of the one or more inorganic acids to the one or more organic acids includes for example 10/1 to 1/10, 9/1 to 1/9, 8/1 to 1/8, 7/1 to 1/7, 6/1 to 1/6, 5/1 to 1/5, 4/1 to 1/4, 3/1 to 1/3, 2/1 to 1/2, and 1/1. An inorganic acid to organic acid weight ratio of 9/2 to 2/9, 8/3 to 3/8, 7/4 to 4/7, 6/5 to 5/6, and 1/1 is preferred. An inorganic acid to organic acid weight ratio of 1/1 is particularly preferred. The acid is preferably a mixture of hydrochloric acid and acetic acid in a weight ratio of 3/1 to 1/3, 2/1 to 1/2, or 1/1.

The present invention provides a collagen-cellulose material comprising: 3.0-7.0 wt. % of a collagen, based on a total weight of the collagen-cellulose material; 0.8-2.0 wt. % of cellulose or a derivative thereof, based on the total weight of the collagen-cellulose material; 1.0-6.0 wt. % of at least one acid selected from an inorganic acid, an organic acid, and mixtures thereof, based on the total weight of the collagen-cellulose material; and water.

An exemplary aspect of the present invention is to provide a collagen-cellulose material comprising: 3.0-7.0 wt. % of a collagen, based on a total weight of the collagen-cellulose material; 0.8-2.0 wt. % of cellulose or a derivative thereof, based on the total weight of the collagen-cellulose material; 1.0-6.0 wt. % of at least one acid selected from an inorganic acid, an organic acid, and mixtures thereof, based on the total weight of the collagen-cellulose material; and a balance being water, wherein the total weight of the collagen, the cellulose or a derivative thereof, the at least one acid, and water is 100 wt. %.

The collagen-cellulose material may further comprise one or more compounds. Non-limiting examples of the one or more compounds include for example pectin, sodium citrate, ammonium sulfate, citric acid, a crosslinking agent (e.g., glutaraldehyde and/or hydroxyacetaldehyde), an aluminum salt, a sugar, a sugar derivative obtained from a caramelization process, a sugar mixture comprising a sugar or a derivative thereof and a crosslinking agent (e.g., MAILLOSE^{®}), a browning agent, glycerin, an antimicrobial agent (e.g., sodium benzoate and/or hypochlorite), mineral oil, poly(lactic-co-glycolic acid) (PLGA), hydroxyapatite, a surfactant, a plasticizer, a dye, and mixtures thereof.

The one or more compounds are preferably selected from pectin, sodium citrate, ammonium sulfate, citric acid, a crosslinking agent (e.g., glutaraldehyde and/or hydroxyacetaldehyde), an aluminum salt, a sugar, a sugar derivative obtained from a caramelization process, a sugar mixture comprising a sugar or a derivative thereof and a crosslinking agent (e.g., MAILLOSE^{®}), a browning agent, glycerin, sodium benzoate, and mixtures thereof.

The one or more compounds may be present in the collagen-cellulose material in an individual or combined amount of 0.1-25 wt. %, including for example 1.0-25.0 wt. %, 1.5-24.5 wt. %, 2.0-24.0 wt. %, 2.5-23.5 wt. %, 3.0-23.0 wt. %, 3.5-22.5 wt. %, 4.0-22.0 wt. %, 4.5-21.5 wt. %, 5.0-21.0 wt. %, 5.5-20.5 wt. %, 6.0-20.0 wt. %, 6.5-19.5 wt. %, 7.0-19.0 wt. %, 7.5-18.5 wt. %, 8.0-18.0 wt. %, 8.5-17.5 wt. %, 9.0-17.0 wt. %, 9.5-16.5 wt. %, 10.0-16.0 wt. %, 10.5-15.5 wt. %, 11.0-15.0 wt. %, 11.5-14.5 wt. %, 12.0-14.0 wt. %,12.5-13.5 wt. %, 13.0 wt. %, based on a total weight of the collagen-cellulose material.

Pectin may be present in an amount 0.1-5.0 wt. %, including for example 0.5-4.5 wt. %, 1.0-4.0 wt. %, 1.5-3.5 wt. %, 2.0-3.0 wt. %, or 2.5 wt. %, based on a total weight of the collagen-cellulose material.

The crosslinking agent (e.g., glutaraldehyde and/or hydroxyacetaldehyde) may be present in an amount of 50-500 ppm, including for example 100-450 ppm, 150-400 ppm, 200-350 ppm, or 250-300 ppm, based on a total weight of the collagen-cellulose material. Applicants have discovered that when pectin and/or an aluminum salt is combined with a crosslinking agent, crosslinking is enhanced and the hardness of the collagen hollow tubes and the collagen sheets more closely resembles that of blood vessels and tissue, respectively. Applicants have also discovered that when pectin and/or an aluminum salt is/are combined with a crosslinking agent and hydroxyapatite, crosslinking is enhanced and the hardness of the collagen solid more closely resembles that of bone.

The aluminum salt may be one or more aluminum salts selected from aluminum acetate, aluminum acetotartrate, aluminum acetylacetonate, aluminum bis(acetylsalicylate), aluminum borate, aluminum bromate, aluminum bromide, aluminum chlorate, aluminum chloride, aluminum citrate, aluminum fluoride, aluminum formate, aluminum iodide, aluminum lactate, aluminum maleate, aluminum nitrate, aluminum octoate, aluminum oleate, aluminum palmitate, aluminum phosphate, aluminum salicylate, aluminum stearate, aluminum sulfate, aluminum tartrate, aluminum tri(sec-butoxide), and mixtures thereof.

The sugar may be one or more sugars selected from a monosaccharide, a disaccharide, and mixtures thereof. Non-limiting examples of the monosaccharide include glucose, fructose, galactose, ribose, arabinose, xylose, lyxose, allose, altrose, mannose, gulose, iodose, talose, and mixtures thereof. Non-limiting examples of the disaccharide include sucrose, lactulose, lactose, maltose, cellobiose, kojibiose, nigerose, isomaltose, trehalose, α,α-trehalose, β,β-trehalose, α,β-trehalose, sophorose, laminaribiose, gentiobiose, turanose, maltulose, palatinose, gentiobiose, mannobiose, melibiose, rutinose, rutinulose, xylobiose, and mixtures thereof.

The sugar derivative obtained from a caramelization process may be present in a sugar mixture further comprising a crosslinking agent (e.g., hydroxyacetaldehyde, a.k.a., glycolaldehyde). MAILLOSE^{®} is a particularly preferred sugar mixture and/or browning agent.

Applicants have discovered that the specific composition of the collagen-cellulose material can be adjusted (e.g., increasing or decreasing the concentration of one or more compounds within the collagen-cellulose material including, but not limited to, pectin, glutaraldehyde, aluminum, sugar, hydroxyapetite) in order to exhibit a desired tensile strength, modulus of elasticity, flexibility, porosity, compressibility, decompressibility (e.g., an ability to return to at least 80% of the original position), hardness, degree of crosslinking, wetness, sealability (e.g., an ability to maintain a hermetic seal following exposure to heat and/or pressure), and/or stability (e.g., a shelf life of at least six months with no change in physicochemical properties), as well as to mimic specific features of a desired anatomical structure.

The collagen-cellulose material may comprise less than 1.0 wt. % of calcium or a calcium compound (e.g., calcium oxide (a.k.a., lime), or calcium hydroxide (a.k.a., hydrated lime, caustic lime, slaked lime)), including for example less than 0.5 wt. %, less than 0.1 wt. %, less than 0.01 wt. %, and less than 0.001 wt. %, based on a total weight of the collagen-cellulose material. The collagen-cellulose material is particularly preferably decalcified and thus does not comprise calcium or a calcium compound.

The present invention provides a collagen-cellulose material comprising: 3.0-7.0 wt. % of a collagen, based on a total weight of the collagen-cellulose material; 0.8-2.0 wt. % of cellulose or a derivative thereof, based on the total weight of the collagen-cellulose material; 1.0-6.0 wt. % of at least one acid selected from an inorganic acid, an organic acid, and mixtures thereof, based on the total weight of the collagen-cellulose material; 0.1-25 wt. % of one or more compounds selected from pectin, sodium citrate, ammonium sulfate, citric acid, a crosslinking agent (e.g., glutaraldehyde and/or hydroxyacetaldehyde), an aluminum salt, a sugar, a sugar derivative obtained from a caramelization process, a sugar mixture comprising a sugar or a derivative thereof and a crosslinking agent (e.g., MAILLOSE^{®}), a browning agent, glycerin, an antimicrobial agent (e.g., sodium benzoate and/or hypochlorite), mineral oil, poly(lactic-co-glycolic acid) (PLGA), hydroxyapatite, a surfactant, a plasticizer, a dye, and mixtures thereof, based on the total weight of the collagen-cellulose material; and water.

The present invention provides a collagen-cellulose material comprising: 3.0-7.0 wt. % of a collagen, based on a total weight of the collagen-cellulose material; 0.8-2.0 wt. % of cellulose or a derivative thereof, based on the total weight of the collagen-cellulose material; 1.0-6.0 wt. % of at least one acid selected from an inorganic acid, an organic acid, and mixtures thereof, based on the total weight of the collagen-cellulose material; 0.1-25 wt. % of one or more compounds selected from pectin, sodium citrate, ammonium sulfate, citric acid, a crosslinking agent (e.g., glutaraldehyde and/or hydroxyacetaldehyde), an aluminum salt, a sugar, a sugar derivative obtained from a caramelization process, a sugar mixture comprising a sugar or a derivative thereof and a crosslinking agent (e.g., MAILLOSE^{®}), a browning agent, glycerin, an antimicrobial agent (e.g., sodium benzoate and/or hypochlorite), mineral oil, poly(lactic-co-glycolic acid) (PLGA), hydroxyapatite, a surfactant, a plasticizer, a dye, and mixtures thereof, based on the total weight of the collagen-cellulose material; and a balance being water, wherein the total weight of the collagen, the cellulose or a derivative thereof, the at least one acid, the one or more compounds, and water is 100 wt. %.

The collagen-cellulose material may be in the form of a hollow tube that mimics a blood vessel (e.g., an artery, a vein, or a capillary), a sheet (e.g., a slab, a pad, a membrane, or a film) that mimics a tissue (e.g., connective tissue, epidermal tissue, muscle tissue, or nerve tissue), or a solid that mimics a bone (e.g., femur bone, tooth). The hollow tube, the sheet, and the solid may also have a desired tensile strength, modulus of elasticity, flexibility, porosity, compressibility, decompressibility, hardness, degree of crosslinking, wetness, sealability, and/or stability.

The hollow tube may have any desired dimension, including any desired length, inner diameter, and/or outer diameter. Although the hollow tube generally has a circular cross-sectional shape, the hollow tube may have any desired cross-sectional shape including for example irregular, elliptical, triangular, square, pentagonal, hexagonal, etc. The hollow tube may be in the form of a bundle of a plurality of hollow tubes. The hollow tube may have any desired length including for example from 1.0 cm to 100 m. The hollow tube has an internal and/or external diameter from 0.10 mm to 50.0 mm. The hollow tube may represent an artificial blood vessel including an artificial artery, an artificial vein, or an artificial capillary.

The sheet may have any desired dimension, including any desired length, width and/or thickness. The sheet may have a rectangular cross-sectional shape of any desired dimension. The sheet may have any desired length, width and/or thickness including for example from 0.10 mm to 100 m. The sheet may represent an artificial tissue including an artificial connective tissue (e.g., an artificial tendon, an artificial ligament, or an artificial organ), epidermal tissue (e.g., artificial skin), muscle tissue, or nerve tissue.

The solid may have any desired dimension, including any desired shape, length, width, thickness, and/or outer diameter. The solid is generally in the shape of a bone (e.g., femur bone, tooth) and thus may represent an artificial bone.

The present invention also provides a process for producing the collagen-cellulose material, which may be in the form of a hollow tube, a sheet, or a solid. The hollow tubes, sheets, and solid of the collagen-cellulose material of the present invention may be prepared by a variation of a procedure used for the fabrication of collagen casings.

The collagen may be extracted/isolated from any source (e.g., any animal sources) including for example human, equine, bovine, porcine, alligator, and/or fish. The collagen is preferably extracted from bovine, porcine, and/or fish due to reduced antigenicity or an absence of antigenicity when the collagen-cellulose material is introduced *in vivo.*

In an exemplary aspect of the present invention, the collagen is extracted/isolated from the epidermis and/or the corium/dermis layer, which is the fibrous inner layer of the skin just beneath the epidermis and consists essentially of an enriched-collagen tissue, of an animal hide, preferably a bovine or porcine animal hide.

The extracted/isolated collagen may be subjected to a limed split process which involves exposing the extracted/isolated collagen to calcium or a calcium compound (e.g., calcium oxide (a.k.a., lime), or calcium hydroxide (a.k.a., hydrated lime, caustic lime, slaked lime)) to breakdown the collagen into a fibrous structure to obtain a collagen split.

The collagen split may be subjected to decalcification with washing to remove undesirable calcium or calcium compounds, which have been incorporated into the collagen split during the limed split process, to obtain a purified collagen tissue, composed primarily of collagen fibers and collagen fibrils. Extensive washing may be carried out to remove a higher concentration of undesirable calcium or calcium compounds to thereby obtain collagen tissue having a higher degree of purity.

The purified collagen tissue may be subjected to acidification with a buffer composition, which comprises citric acid and/or a salt thereof (e.g., sodium citrate), ammonium sulfate, and water, to obtain a collagen material at a pH of 3.0-6.0, including for example 3.5-5.5, 4.0-5.0, or 4.5, preferably 4.0-5.0, 4.1-4.9, 4.2-4.8, 4.3-4.7, 4.4-4.6, or 4.5, and most preferably 4.5.

The collagen material may be cut and/or ground, optionally at a temperature of 10-25°C, including for example 11-24°C, 12-23°C, 13-22°C, 14-21°C, 15-20°C, 16-19°C, or 17-18°C, preferably 11-19°C, 14-18°C, 13-17°C, 12-16°C, or 13-15°C, and most preferably 13-17°C, 12-16°C, or 13-15°C, to obtain collagen which may be in the form of a powder, a fibril, and/or a fiber.

The collagen is mixed with: cellulose or a derivative thereof; at least one acid selected from an inorganic acid, an organic acid, and mixtures thereof; one or more optional compounds (e.g., a crosslinking agent (e.g., glutaraldehyde) and/or an aluminum salt); and water, for a time period of approximately 10-20 minutes, including for example 11-19 minutes, 12-18 minutes, 13-17 minutes, 14-16 minutes, or preferably 15 minutes, to form a fibrous slurry or a doughy mass having a pH of less than or equal to 3.0, including for example 1.0-3.0, 1.5-2.5, or 2.0.

Important steps in the formation of a fibrous slurry or a doughy mass having an appropriate consistency include the specific nature and concentrations of the components, the temperature, the pH, and/or the physical mixing/blending of the components.

The fibrous slurry or the doughy mass may be subjected to one or more treatments selected from cutting, grinding, homogenization, filtration (e.g., through a 0.008 mesh screen and/or a 0.006 mesh screen), dipping, soaking and/or extrusion (e.g., in the presence of an aluminum salt solution and/or a sugar mixture comprising a sugar or a derivative thereof and a crosslinking agent (e.g., MAILLOSE^{®})), washing (e.g., washing with an aqueous solution which may comprise an optional crosslinking agent, such as glutaraldehyde, for example), and/or drying.

The aluminum salt solution for use in the dipping, soaking and/or extrusion may comprise one or more of the aforementioned aluminum salts and an optional crosslinking agent (e.g., glutaraldehyde). A non-limiting example of the aluminum salt solution comprises 2.9-3.5 wt. % of an ammonium salt, 1.0-1.6 wt. % of ammonium sulfate, 0.4-1.0 wt. % of citric acid, 0.05-0.15 wt. % of ammonium hydroxide, and 225-275 ppm of glutaraldehyde. The fibrous slurry or the doughy mass may be soaked for a period of time (e.g., 30 minutes to 3.5 hours) in the aluminum salt solution prior to extrusion and/or be exposed to the aluminum salt solution during dipping and/or extrusion.

The sugar mixture comprising a sugar or a derivative thereof and a crosslinking agent (e.g., MAILLOSE^{®}) may further comprise glycerin/glycerol and water. The fibrous slurry or the doughy mass may be soaked for a period of time (e.g., 30 minutes to 3.5 hours) in the sugar mixture prior to extrusion and/or be exposed to the sugar mixture during dipping and/or extrusion.

Following the treatment, the fibrous slurry or the doughy mass is subjected to aging in a storage tank at a temperature of less than 25°C for a period of time (e.g., at least 6, 7, 8, 9, 10, 11, or 12 hours) sufficient to obtain a collagen-cellulose gel which is maintained at a temperature of less than 25°C prior to being subjected to an extrusion process to obtain a collagen-cellulose material. The aluminum salt solution and/or the sugar mixture may be added to the storage tank at various concentrations in order to obtain a desired degree of crosslinking prior to being subjected to the extrusion process. In addition, or as an alternative, to adding the aluminum salt solution and/or the sugar mixture to the storage tank, the collagen-cellulose gel may be soaked for a period of time (e.g., 30 minutes to 3.5 hours) in the aluminum salt solution and/or the sugar mixture prior to extrusion and/or be exposed to the aluminum salt solution and/or the sugar mixture during extrusion.

The collagen-cellulose material obtained from the extrusion may be of any desired shape, length, width, thickness, inner diameter, and/or outer diameter, and may be in the form of a hollow tube, a sheet, or a solid depending on the specific type of extruder utilized during the extrusion process and the specific extrusion conditions (e.g., temperature, pressure, rate of extrusion, etc.). The collagen-cellulose material may be subjected to optional drying after the extrusion to obtain a desired moisture content and degree of wetness. The composition and manufacturing conditions of collagen-cellulose material is adjusted and designed/fabricated to exhibit a desired tensile strength, modulus of elasticity, flexibility, porosity, compressibility, decompressibility (e.g., an ability to return to at least 80% of the original position), hardness, degree of crosslinking, wetness, sealability (e.g., an ability to maintain a hermetic seal following exposure to heat and/or pressure), and/or stability (e.g., a shelf life of at least six months with no change in physicochemical properties), as well as to mimic specific features of a desired anatomical structure.

The following Table is a non-limiting comparison of various physicochemical properties of the collagen-cellulose material in the form of a hollow tube, a sheet, and a solid.

**Table**

| PROPERTY | TUBE | SHEET | SOLID |
|---|---|---|---|
| Tensile strength | Particularly preferred | Particularly preferred | Acceptable |
| Modulus of elasticity | Particularly preferred | Acceptable | Acceptable |
| Flexibility | Particularly preferred | Acceptable | Acceptable |
| Porosity | Particularly preferred | Acceptable | Acceptable |
| Compressibility | Particularly preferred | Particularly preferred | Acceptable |
| Decompressibility | Particularly preferred | Particularly preferred | Acceptable |
| Hardness | Acceptable | Particularly preferred | Acceptable |
| Degree of crosslinking | Preferred | Particularly preferred | Acceptable |
| Wetness | Acceptable | Acceptable | Acceptable |
| Sealability | Particularly preferred | Particularly preferred | Acceptable |
| | | | |
| Stability | Particularly preferred | Particularly preferred | Particularly preferred |

The collagen-cellulose material obtained from the process of the present invention comprises: 1.0-9.0 wt. % of a collagen, based on a total weight of the collagen-cellulose material; 0.2-3.0 wt. % of cellulose or a derivative thereof, based on the total weight of the collagen-cellulose material; 0.5-6.5 wt. % of at least one acid selected from an inorganic acid, an organic acid, and mixtures thereof, based on the total weight of the collagen-cellulose material; and water.

The collagen-cellulose material obtained from the process of the present invention may be kept wet in a solution comprising: 5-15 wt. %, preferably 10 wt. % of glycerin; and 0.01-1.0 wt. %, preferably 0.1 wt. % of sodium benzoate.

The extracted/isolated collagen, the collagen-cellulose gel, and the collagen-cellulose material which may be in the form of a hollow tube, a sheet, or a solid, may be characterized by gel electrophoresis, capillary electrophoresis, and/or high-performance liquid chromatography, which may be carried out in the presence of sodium dodecyl sulfate. The degree of cross-linking of the collagen-cellulose gel and the collagen-cellulose material can also be determined.

The present invention also provides an artificial blood vessel including an artificial artery, an artificial vein, or an artificial capillary comprising the collagen-cellulose material which is in the form of a hollow tube.

The present invention also provides an artificial tissue including an artificial connective tissue (e.g., an artificial tendon, an artificial ligament, or an artificial organ), an artificial epidermal tissue (e.g., artificial skin), an artificial muscle tissue, or an artificial nerve tissue comprising the collagen-cellulose material which is in the form of a sheet.

The present invention also provides an artificial bone comprising the collagen-cellulose material which is in the form of a solid.

The present invention also provides a medical device comprising the collagen-cellulose material for testing phlebotomical, surgical or orthopedic instrumentation during research and development and/or for practicing phlebotomical, surgical or orthopedic procedures during the training of healthcare professionals (e.g., physicians, phlebotomists, surgeons, and medical students).

The medical device may further comprise a synthetic polymer, a natural polymer, an elastomer, a plastic, a thermoplastic, a synthetic rubber, a natural rubber, a silicone rubber, a hydrogel, a ceramic, a metal, an adhesive, a cement, wood, a dye, a pigment, an actual human blood vessel, bone or tissue, an actual animal blood vessel, bone or tissue, and combinations thereof.

The medical device comprising the collagen-cellulose material may be in the form of an artificial blood vessel (e.g., an artificial artery, an artificial vein, or an artificial capillary), an artificial bone (e.g., femur bone, tooth), or an artificial tissue including an artificial connective tissue (e.g., an artificial tendon, an artificial ligament, or an artificial organ), an artificial epidermal tissue (e.g., artificial skin), an artificial muscle tissue, or an artificial nerve tissue.

The medical device may further comprise a support member for supporting the artificial blood vessel, the artificial bone, and/or the artificial tissue. Non-limiting examples of the support member include a mannequin arm or an artificial arm for supporting the artificial blood vessel, a stand for supporting the artificial bone, and/or a tray for supporting the artificial tissue.

An exemplary aspect of the present invention is to provide a medical device comprising the collagen-cellulose material in the form of an artificial blood vessel, and a support member that may be in the form of a mannequin arm or an artificial arm for supporting the artificial blood vessel, which is useful for practicing phlebotomical procedures during the training of healthcare professionals (e.g., physicians, phlebotomists, surgeons, and medical students).

The medical device comprising the collagen-cellulose material of the present invention has a number of advantages including obviating the need to test or practice phlebotomical, surgical or orthopedic instrumentation and procedures on animals and human cadavers.

Disclosed herein, but not claimed, is a method for testing phlebotomical, surgical or orthopedic instrumentation and/or practicing a phlebotomical, surgical or orthopedic procedure comprising performing testing of the phlebotomical, surgical or orthopedic instrumentation or practicing of the phlebotomical, surgical or orthopedic procedure on a medical device comprising the collagen-cellulose material of the present invention.

The present invention also provides an article or device for *in vitro* or *in vivo* use comprising the collagen-cellulose material for pharmaceutical, medicinal, therapeutic, phlebotomical, surgical, or orthopedic applications.

The article for *in vitro* or *in vivo* use may further comprise a drug (e.g., antibiotic, analgesic, antihistamine, stimulant, tranquilizer, narcotic, antidepressant, hormone, anticoagulant, laxative, diuretic, etc.) and/or a pharmaceutically acceptable excipient. The article may be in any form including for example a powder, a pill, a tablet, a capsule, a liquid, a solution, a suspension, a slurry, a drink, a syrup, a thin film or membrane, a bandage, a transdermal patch, a suppository, a cream, a gel, a liniment, a balm, a lotion, an ointment, and a salve.

The device (e.g., an orthopedic device) for *in vitro* or *in vivo* use comprises the collagen-cellulose material in a form selected from a prosthetic blood vessel (e.g., a prosthetic artery, a prosthetic vein, or a prosthetic capillary), a prosthetic bone (e.g., a prosthetic femur bone, a prosthetic tooth), and/or a prosthetic tissue including prosthetic connective tissue (e.g., prosthetic tendon, prosthetic ligament, or prosthetic organ), prosthetic epidermal tissue (e.g., prosthetic skin), prosthetic muscle tissue, and/or prosthetic nerve tissue.

The collagen-cellulose material which is in the form of a hollow tube may be useful as a guide, a stent protector, a nerve protector, and for various biochemical mechanisms.

## Claims

1. A collagen-cellulose material comprising:
1.0-9.0 wt. % of a collagen, based on a total weight of the collagen-cellulose material;
0.2-3.0 wt. % of cellulose or a derivative thereof, based on the total weight of the collagen-cellulose material;
0.5-6.5 wt. % of at least one acid selected from the group consisting of an inorganic acid, an organic acid, and mixtures thereof, based on the total weight of the collagen-cellulose material; and
water,
wherein the collagen-cellulose material is in a form of an artificial blood vessel having an internal and/or external diameter including from 0.1 to 50.0 mm, an artificial tissue, and/or an artificial bone.

2. The collagen-cellulose material of claim 1, further comprising at least one component selected from the group consisting of a synthetic polymer, a natural polymer, an elastomer, a plastic, a thermoplastic, a synthetic rubber, a natural rubber, a silicone rubber, a hydrogel, a ceramic, a metal, an adhesive, a cement, wood, a dye, a pigment, an actual human blood vessel, bone or tissue, an actual animal blood vessel, bone or tissue, and combinations thereof.

3. The collagen-cellulose material of claim 1 or 2, further comprising a support member in the form of a mannequin arm or an artificial arm for supporting the artificial blood vessel.

4. The collagen-cellulose material of claim 1 or 2, which is configured to practice a phlebotomical procedure.

5. The collagen-cellulose material of any one of the preceding claims, wherein the collagen-cellulose material comprises:
3.0-7.0 wt. % of the collagen;
0.8-2.0 wt. % of the cellulose or the derivative thereof; and
1.0-6.0 wt. % of the at least one acid.

6. The collagen-cellulose material of any one of the preceding claims, wherein the cellulose is present and in the collagen-cellulose material is obtained from wood, and/or at least one pectin-rich plant material selected from the group consisting of a peel of a citrus fruit, apple pomace, sugar beet pulp, a sunflower head, a carrot, a potato, a tomato, and combinations thereof.

7. The collagen-cellulose material of any one of the preceding claims, wherein the derivative of cellulose is present in the collagen-cellulose material and is at least one ether derivative of cellulose selected from the group consisting of methylcellulose, ethylcellulose, ethylmethylcellulose, hydroxyethylcellulose, hydroxyethylmethylcellulose, hydroxypropylcellulose, hydroxypropylmethylcellulose, ethylhydroxyethylcellulose, carboxymethylcellulose, carboxyethylcellulose, and mixtures thereof.

8. The collagen-cellulose material of any one of the preceding claims, wherein the derivative of cellulose is present in the collagen-cellulose material and is at least one ester derivative of cellulose selected from the group consisting of cellulose acetate, cellulose triacetate, cellulose propionate, cellulose acetate propionate, cellulose acetate butyrate, cellulose nitrate, cellulose sulfate, and mixtures thereof

9. The collagen-cellulose material of any one of the preceding claims, wherein the acid is one or more inorganic acids selected from the group consisting of hydrochloric acid, nitric acid, phosphoric acid, sulfuric acid, boric acid, hydrofluoric acid, hydrobromic acid, perchloric acid, and mixtures thereof.

10. The collagen-cellulose material of any one of the preceding claims, wherein the acid is one or more organic acids selected from the group consisting of formic acid, acetic acid, propionic acid, butyric acid, valeric acid, caproic acid, and mixtures thereof.

11. The collagen-cellulose material of any one of the preceding claims, wherein the acid is a mixture of one or more inorganic acids and one or more organic acids, wherein a weight ratio of the one or more inorganic acids to the one or more organic acids is 10/1 to 1/10.

12. The collagen-cellulose material of any one of the preceding claims, wherein the acid is a mixture of hydrochloric acid and acetic acid in a weight ratio of 3/1 to 1/3.

13. The collagen-cellulose material of any one of the preceding claims, wherein the collagen-cellulose material further comprises 0.1-25 wt. % of one or more compounds selected from the group consisting of pectin, sodium citrate, ammonium sulfate, citric acid, a crosslinking agent, an aluminum salt, a sugar, a sugar derivative obtained from a caramelization process, a sugar mixture, a browning agent, glycerin, an antimicrobial agent, hydroxyapatite, and mixtures thereof, based on the total weight of the collagen-cellulose material.

14. The collagen-cellulose material of claim 4, wherein the support member is in the form of a mannequin arm or an artificial arm for supporting the artificial blood vessel.

## Patentansprüche

1. Kollagen-Cellulose-Material, umfassend:
1,0-9,0 Gew.-% eines Kollagens, bezogen auf das Gesamtgewicht des Kollagen-Cellulose-Materials;
0,2-3,0 Gew.-% Cellulose oder eines Derivats davon, bezogen auf das Gesamtgewicht des Kollagen-Cellulose-Materials;
0,5-6,5 Gew.-% mindestens einer Säure, ausgewählt aus der Gruppe bestehend aus einer anorganischen Säure, einer organischen Säure und Mischungen davon, bezogen auf das Gesamtgewicht des Kollagen-Cellulose-Materials; und
Wasser,
wobei das Kollagen-Cellulose-Material in Form eines künstlichen Blutgefäßes mit einem inneren und/oder äußeren Durchmesser einschließlich von 0,1 bis 50,0 mm, eines künstlichen Gewebes und/oder eines künstlichen Knochens vorliegt.

2. Kollagen-Cellulose-Material nach Anspruch 1, das ferner mindestens eine Komponente umfasst, ausgewählt aus der Gruppe bestehend aus einem synthetischen Polymer, einem natürlichen Polymer, einem Elastomer, einem Kunststoff, einem Thermoplast, einem synthetischen Kautschuk, einem natürlichen Kautschuk, einem Silikonkautschuk, einem Hydrogel, einer Keramik, einem Metall, einem Klebstoff, einem Zement, Holz, einem Farbstoff, einem Pigment, einem tatsächlichen menschlichen Blutgefäß, Knochen oder Gewebe, einem tatsächlichen tierischen Blutgefäß, Knochen oder Gewebe und Kombinationen davon.

3. Kollagen-Cellulose-Material nach Anspruch 1 oder 2, umfassend ferner ein Stützelement in Form eines Mannequin-Arms oder eines künstlichen Arms zum Stützen des künstlichen Blutgefäßes.

4. Kollagen-Cellulose-Material nach Anspruch 1 oder 2, das für die Durchführung eines phlebotomischen Verfahrens konfiguriert ist.

5. Kollagen-Cellulose-Material nach einem der vorherigen Ansprüche, wobei das Kollagen-Cellulose-Material umfasst:
3,0-7,0 Gew.-% des Kollagens;
0,8-2,0 Gew.-% der Cellulose oder des Derivats davon; und
1,0-6,0 Gew.-% der mindestens einen Säure.

6. Kollagen-Cellulose-Material nach einem der vorherigen Ansprüche, wobei die Cellulose vorhanden ist und in dem Kollagen-Cellulose-Material aus Holz und/oder mindestens einem pektinreichen Pflanzenmaterial, ausgewählt aus der Gruppe bestehend aus der Schale einer Zitrusfrucht, Apfeltrester, Zuckerrübenschnitzel, einem Sonnenblumenkopf, einer Karotte, einer Kartoffel, einer Tomate und Kombinationen davon, gewonnen wird.

7. Kollagen-Cellulose-Material nach einem der vorherigen Ansprüche, wobei das Cellulose-Derivat in dem Kollagen-Cellulose-Material vorhanden ist und mindestens ein Ether-Derivat von Cellulose ist, ausgewählt aus der Gruppe bestehend aus Methylcellulose, Ethylcellulose, Ethylmethylcellulose, Hydroxyethylcellulose, Hydroxyethylmethylcellulose, Hydroxypropylcellulose, Hydroxypropylmethylcellulose, Ethylhydroxyethylcellulose, Carboxymethylcellulose, Carboxyethylcellulose und Mischungen davon.

8. Kollagen-Cellulose-Material nach einem der vorherigen Ansprüche, wobei das Cellulose-Derivat in dem Kollagen-Cellulose-Material vorhanden ist und mindestens ein Ester-Derivat von Cellulose ist, ausgewählt aus der Gruppe bestehend aus Celluloseacetat, Cellulosetriacetat, Cellulosepropionat, Celluloseacetatpropionat, Celluloseacetatbutyrat, Cellulosenitrat, Cellulosesulfat und Mischungen davon.

9. Kollagen-Cellulose-Material nach einem der vorherigen Ansprüche, wobei die Säure eine oder mehrere anorganische Säuren ist, ausgewählt aus der Gruppe bestehend aus Salzsäure, Salpetersäure, Phosphorsäure, Schwefelsäure, Borsäure, Fluorwasserstoffsäure, Bromwasserstoffsäure, Perchlorsäure und Mischungen davon.

10. Kollagen-Cellulose-Material nach einem der vorherigen Ansprüche, wobei die Säure eine oder mehrere organische Säuren ist, ausgewählt aus der Gruppe bestehend aus Ameisensäure, Essigsäure, Propionsäure, Buttersäure, Valeriansäure, Capronsäure und Mischungen davon.

11. Kollagen-Cellulose-Material nach einem der vorherigen Ansprüche, wobei die Säure eine Mischung aus einer oder mehreren anorganischen Säuren und einer oder mehreren organischen Säuren ist, wobei das Gewichtsverhältnis der einen oder mehreren anorganischen Säuren zu der einen oder mehreren organischen Säuren 10/1 bis 1/10 beträgt.

12. Kollagen-Cellulose-Material nach einem der vorherigen Ansprüche, wobei die Säure ein Gemisch aus Salzsäure und Essigsäure in einem Gewichtsverhältnis von 3/1 bis 1/3 ist.

13. Kollagen-Cellulose-Material nach einem der vorherigen Ansprüche, wobei das Kollagen-Cellulose-Material ferner 0,1-25 Gew.-% einer oder mehrerer Verbindungen umfasst, ausgewählt aus der Gruppe bestehend aus Pektin, Natriumcitrat, Ammoniumsulfat, Citronensäure, einem Vernetzungsmittel, einem Aluminiumsalz, einem Zucker, einem Zuckerderivat, das aus einem Karamellisierungsverfahren erhalten wurde, einer Zuckermischung, einem Bräunungsmittel, Glycerin, einem antimikrobiellen Mittel, Hydroxyapatit und Mischungen davon, bezogen auf das Gesamtgewicht des Kollagen-Cellulose-Materials.

14. Kollagen-Cellulose-Material nach Anspruch 4, wobei das Stützelement die Form eines Mannequin-Arms oder eines künstlichen Arms zum Stützen des künstlichen Blutgefäßes hat.

## Revendications

1. Matériau à base de collagène-cellulose comprenant :
1,0-9,0 % en poids d'un collagène, par rapport au poids total du matériau à base de collagène-cellulose ;
0,2-3,0 % en poids de cellulose ou d'un dérivé de celle-ci, par rapport au poids total du matériau à base de collagène-cellulose ;
0,5-6,5 % en poids d'au moins un acide choisi dans le groupe constitué par un acide inorganique, un acide organique et leurs mélanges, par rapport au poids total de la matière à base de collagène-cellulose ; et
de l'eau,
dans lequel le matériau à base de collagène-cellulose est sous la forme d'un vaisseau sanguin artificiel ayant un diamètre interne et/ou externe compris entre 0,1 et 50,0 mm, d'un tissu artificiel et/ou d'un os artificiel.

2. Matériau à base de collagène-cellulose selon la revendication 1, comprenant en outre au moins un composant choisi dans le groupe constitué d'un polymère synthétique, d'un polymère naturel, d'un élastomère, d'un plastique, d'un thermoplastique, d'un caoutchouc synthétique, d'un caoutchouc naturel, d'un caoutchouc de silicone, d'un hydrogel, d'une céramique, d'un métal, d'un adhésif, d'un ciment, de bois, d'un colorant, d'un pigment, d'un vaisseau sanguin, d'un os ou d'un tissu humain réel, d'un vaisseau sanguin, d'un os ou d'un tissu animal réel, et de leurs combinaisons.

3. Matériau à base de collagène-cellulose selon la revendication 1 ou 2, comprenant en outre un élément de support sous la forme d'un bras de mannequin ou d'un bras artificiel pour supporter le vaisseau sanguin artificiel.

4. Matériau à base de collagène-cellulose selon la revendication 1 ou 2, qui est configuré pour pratiquer une procédure phlébotomique.

5. Matériau à base de collagène-cellulose selon l'une quelconque des revendications précédentes, dans lequel le matériau à base de collagène-cellulose comprend :
3,0-7,0 % en poids de collagène,
0,8-2,0 % en poids de cellulose ou de son dérivé ; et
1,0-6,0 % en poids dudit au moins un acide.

6. Matériau à base de collagène-cellulose selon l'une quelconque des revendications précédentes, dans lequel la cellulose est présente et dans le matériau à base de collagène-cellulose est obtenue à partir de bois, et/ou d'au moins un matériau végétal riche en pectine choisi dans le groupe constitué par une peau d'agrume, un marc de pomme, une pulpe de betterave à sucre, une tête de tournesol, une carotte, une pomme de terre, une tomate, et leurs combinaisons.

7. Matériau à base de collagène-cellulose selon l'une quelconque des revendications précédentes, dans lequel le dérivé de cellulose est présent dans le matériau à base de collagène-cellulose et est au moins un dérivé éther de cellulose choisi dans le groupe constitué par la méthylcellulose, l'éthylcellulose, l'éthylméthylcellulose, l'hydroxyéthylcellulose, l'hydroxyéthylméthylcellulose, l'hydroxypropylméthylcellulose, l'hydroxypropylcellulose, l'éthylhydroxyéthylcellulose, la carboxyméthylcellulose, la carboxyéthylcellulose, et leurs mélanges.

8. Matériau à base de collagène-cellulose selon l'une quelconque des revendications précédentes, dans lequel le dérivé de cellulose est présent dans le matériau à base de collagène-cellulose et est au moins un dérivé ester de cellulose choisi dans le groupe constitué par l'acétate de cellulose, le triacétate de cellulose, le propionate de cellulose, l'acétate propionate de cellulose, l'acétate butyrate de cellulose, le nitrate de cellulose, le sulfate de cellulose, et leurs mélanges.

9. Matériau à base de collagène-cellulose selon l'une quelconque des revendications précédentes, dans lequel l'acide est un ou plusieurs acides inorganiques choisis dans le groupe constitué par l'acide chlorhydrique, l'acide nitrique, l'acide phosphorique, l'acide sulfurique, l'acide borique, l'acide fluorhydrique, l'acide bromhydrique, l'acide perchlorique, et leurs mélanges.

10. Matériau à base de collagène-cellulose selon l'une quelconque des revendications précédentes, dans lequel l'acide est un ou plusieurs acides organiques choisis dans le groupe constitué par l'acide formique, l'acide acétique, l'acide propionique, l'acide butyrique, l'acide valérique, l'acide caproïque, et leurs mélanges.

11. Matériau à base de collagène-cellulose selon l'une quelconque des revendications précédentes, dans lequel l'acide est un mélange d'un ou plusieurs acides inorganiques et d'un ou plusieurs acides organiques, dans lequel un rapport pondéral dudit ou desdits acides inorganiques audit ou auxdits acides organiques est de 10/1 à 1/10.

12. Matériau à base de collagène-cellulose selon l'une quelconque des revendications précédentes, dans lequel l'acide est un mélange d'acide chlorhydrique et d'acide acétique dans un rapport pondéral de 3/1 à 1/3.

13. Matériau à base de collagène-cellulose selon l'une quelconque des revendications précédentes, dans lequel le matériau à base de collagène-cellulose comprend en outre 0,1 à 25 % en poids d'un ou plusieurs composés choisis dans le groupe constitué de la pectine, du citrate de sodium, du sulfate d'ammonium, de l'acide citrique, d'un agent de réticulation, d'un sel d'aluminium, d'un sucre, d'un dérivé du sucre obtenu à partir d'un procédé de caramélisation, d'un mélange de sucres, d'un agent de brunissement, de glycérine, d'un agent antimicrobien, d'hydroxyapatite et de leurs mélanges, par rapport au poids total du matériau à base de collagène-cellulose.

14. Matériau à base de collagène-cellulose selon la revendication 4, dans lequel l'élément de support se présente sous la forme d'un bras de mannequin ou d'un bras artificiel pour supporter le vaisseau sanguin artificiel.
